# EUROPÄISCHE PATENTANMELDUNG

(11) **EP 4 806 979 A1**
(43) Veröffentlichungstag der Anmeldung: **16.09.2026**
(21) Anmeldenummer: 26163201.2
(22) Anmeldetag: 09.03.2026
(51) Int. Cl.: C10L 3/08, C07C 1/12, C07C 9/04

(54) **VERFAHREN UND ANLAGE ZUR HERSTELLUNG VON METHAN AUS EINEM GASGEMISCH UMFASSEND WASSERSTOFF UND KOHLENDIOXID**

(30) Priorität: 10.03.2025 DE 102025108901; 10.03.2025 LU 600510
(71) Anmelder: thyssenkrupp Uhde GmbH, 44141 Dortmund (DE); thyssenkrupp AG, 45143 Essen (DE)
(72) Erfinder: Schulz, Alexander, 60439 Frankfurt (DE); Schirrmeister, Steffen, 45472 Mülheim an der Ruhr (DE)
(74) Vertreter: thyssenkrupp Intellectual Property GmbH

(57) **Zusammenfassung**

Die Erfindung betrifft ein Verfahren zur Herstellung von Methan aus einem Synthesegas. Bei einem Verfahren, bei dem die Reinheit des Produktes Methan erhöht wird, wobei gleichzeitig Kosten gespart werden können, sind mindestens die folgenden Schritte vorgesehen: Verdichten eines Gemisches umfassend Wasserstoff und CO2 mit einem Synthesegasverdichter (2); Zuführen des Gemisches in eine erste Methansynthese (3); Zuführen des entstandenen Produktstroms in einen ersten Hochdruckabscheider (4), wobei Wasser von dem Produktstrom abgeschieden wird; Zuführen des Produktstroms in eine zweite Methansynthese (5); Zuführen des Produktstroms in einen zweiten Hochdruckabscheider (6), wobei Wasser von dem Produktstrom abgeschieden wird; Aufbereitung des Produktstroms zum Erhalt von Methan und eines Recycle-Stroms, wobei der Recycle-Strom in den Prozess zurückgeführt wird.

## Beschreibung

Die Erfindung betrifft ein Verfahren zur Herstellung von Methan aus einem Synthesegas.

Daneben betrifft die Erfindung außerdem eine Anlage zur Herstellung von Methan aus einem Synthesegas.

Synthetisches Erdgas "SNG" (Synthetic Natural Gas), kann mit Hilfe von Power-to-SNG-Verfahren (abgekürzt "P2G") hergestellt werden, bei denen Wasser, CO₂ und elektrische Energie in ein brennbares Gas von ähnlicher Qualität wie Erdgas umgewandelt werden. Um eine zufriedenstellende wirtschaftliche Rentabilität zu erzielen, basiert P2G auf der Nutzung elektrischer Energie aus einer erneuerbaren Quelle in Zeiten, in denen eine Überproduktion dieser Energie besteht.

Folglich erfordert die Methanisierungstechnologie eine große betriebliche Flexibilität in Bezug auf Leistung, Durchsatz und Verarbeitung. In diesem Bereich gibt es mehrere technologische Optionen. Die erste Option besteht in der direkten Co-Elektrolyse von Wasser in Form von Dampf und anthropogenem CO₂ zur Erzeugung von CO und H₂. Diese Umwandlung findet im Allgemeinen bei hohen Temperaturen statt und das erzeugte Gas ist ein Synthesegas, dessen Zusammensetzungsmerkmale denen der Biomassevergasung ähneln (CO, H₂, CO₂ und H₂O). Der Hauptunterschied liegt in der besseren Kontrolle des H₂/CO-Verhältnisses ohne einen Gas-Wasser-Reaktionsschritt, der mit "WGS" (für Water Gas Shift) abgekürzt und als "Dussan-Reaktion" bezeichnet wird. Die Formel für diese WGS-Reaktion lautet CO + H₂O → H₂ + CO₂.

Die zweite Möglichkeit besteht darin, das Wasser durch eine alkalische oder Polymerelektrolytmembran-Elektrolyse umzuwandeln, um H₂ an der Kathode und O₂ an der Anode zu erzeugen. Das so erzeugte H₂ wird dann mit CO₂ vermischt, um Synthesegas zu bilden, das die Quelle des synthetischen Erdgases ist.

Die Herstellung von Methan aus dem Synthesegas basiert auf der katalytischen Methanisierung (oder Hydrierung) des CO oder CO₂, der so genannten "Sabatier-Reaktion". Bei der Methanisierung wird das Kohlenmonoxid oder -dioxid in Gegenwart von Wasserstoff und einem Katalysator, im Allgemeinen auf Basis von Nickel oder einem anderen mehr oder weniger edlen Übergangsmetall, in Methan umgewandelt. Zur Herstellung von SNG aus Synthesegas wird bei der ersten Option eine CO-Methanisierungsreaktion eingesetzt, während bei der zweiten Option eine CO₂-Methanisierungsreaktion verwendet wird.

Die Methanisierungsreaktion ist eine exotherme Reaktion mit einer Verringerung der Molzahl; nach dem Prinzip von Le Chatelier wird die Reaktion durch Erhöhung des Drucks gefördert und durch Erhöhung der Temperatur gehemmt. Diese stark exothermen, ausgeglichenen Reaktionen erfordern eine gute Kontrolle der Kühlung des Reaktors, in dem sie ablaufen. Die Kontrolle der Temperatur im Reaktor und damit die Abfuhr der durch die Reaktion erzeugten Wärme ist einer der Schlüssel zur Minimierung der Deaktivierung des Katalysators durch Versinterung und zur Maximierung der Methanumwandlungsrate.

Einige der Methanisierungsreaktortechnologien verwenden einen dichten Wirbelschichtreaktor, wobei das Bett durch den Katalysator der Methanisierungsreaktion gebildet wird. Die durch die Reaktion erzeugte Wärme wird daher durch Wärmetauscher abgeführt, die in das Wirbelbett eingetaucht sind. Aufgrund der sehr hohen Exothermie der Reaktion ist die abzuführende Wärmemenge und damit die erforderliche Austauschfläche jedoch sehr groß. Daher führt das von diesem Austauscher beanspruchte Volumen zu einer Überdimensionierung des Reaktors und vor allem zu einer komplexeren Konstruktion.

Die Verwendung eines Wirbelbetts ist eine einfache Lösung zur Begrenzung der Reaktionstemperatur. Die Fluidisierung des Katalysators durch das Reaktionsgemisch ermöglicht eine nahezu perfekte Homogenisierung der Temperaturen an allen Punkten der Katalysatorschicht, und der Reaktor kann einem isothermen Reaktor gleichgestellt werden. Die Abfuhr der durch die Reaktion erzeugten Wärme wird durch in das Wirbelbett eingetauchte Wärmetauscher erreicht. Die Wärmeaustauschkoeffizienten zwischen der Wirbelschicht und einer in die Schicht eingetauchten Wand sind sehr hoch und ermöglichen es, die Abmessungen des Austauschers und damit die Gesamtgröße des Reaktors zu minimieren.

In dem Temperaturbereich, der in den Wirbelschicht-Methanisierungsreaktoren verwendet wird, ist die Kinetik der Methanisierungsreaktion sehr schnell, so dass nur eine geringe Menge an Katalysator für die chemische Reaktion erforderlich ist. Die Größe des Reaktors und die Menge des verwendeten Katalysators ergeben sich daher aus den Gesamtabmessungen des im Wirbelbett installierten Austauschers und der erforderlichen Übertragungsfläche.

Das entstehende Methan wird in der Regel in einem Adsorptionsbett "getrocknet". Die Trocknung ist notwendig, um den Wassergehalt auf einen spezifikationsgerechten Wert zu reduzieren. Unter den vorgenannten Bedingungen hergestelltes Methan erfüllt die Anforderungen zur Einspeisung in ein öffentliches Gasnetz.

Für die Trocknung des Gases ist ein Adsorptionsbett vorgesehen, das zur Regeneration mit einem Teilstrom des getrockneten Methans diskontinuierlich durchströmt wird. Dieses Wasser-beladene Methan wird anschließend zum Produktstrom in die Abkühlstrecke nach der zweiten Reaktorstufe zugegeben.

Für andere Anwendungen, wie z.B. einer Verflüssigung von Methan (LNG), werden höhere Anforderungen an die Reinheit des Methans gestellt.

Der Erfindung liegt daher die Aufgabe zugrunde, ein Verfahren sowie eine Anlage zur Herstellung von Methan aus einem Synthesegas bereitzustellen, bei denen die Reinheit des Produktes Methan erhöht wird, wobei gleichzeitig Kosten gespart werden können.

Diese Aufgabe ist bei der vorliegenden Erfindung durch die Merkmale des Patentanspruchs 1 zunächst dadurch gelöst, dass das Verfahren mindestens die folgenden Schritte umfasst: Verdichten eines Gemisches umfassend Wasserstoff und CO₂ mit einem Synthesegasverdichter; Zuführen des Gemisches in eine erste Methansynthese; Zuführen des entstandenen Produktstroms in einen ersten Hochdruckabscheider, wobei Wasser von dem Produktstrom abgeschieden wird; Zuführen des Produktstroms in eine zweite Methansynthese; Zuführen des Produktstroms in einen zweiten Hochdruckabscheider, wobei Wasser von dem Produktstrom abgeschieden wird; Aufbereitung des Produktstroms zum Erhalt von Methan und eines Recycle-Stroms, wobei der Recycle-Strom in den Prozess zurückgeführt wird.

Unter Hochdruck ist im Rahmen der Erfindung ein Druck zu verstehen, der bei der Methanisierung eingestellt wird. Das Hochdruckniveau bei der Methanisierung liegt vorzugsweise im Bereich von 5 bar bis 50 bar Überdruck, bevorzugt zwischen 10 bar und 30 bar Überdruck. Ein zweites Druckniveau, das sogenannte Niederdruckniveau, kann vorzugsweise in einem Bereich zwischen 0,3 und 0,7 bar Überdruck liegen.

Unter einem Synthesegas ist ein Gasgemisch zu verstehen, dass mindestens die Komponenten CO und/oder CO₂ und H₂ enthält. Wird der Feedstrom beispielsweise aus einer Pyrolyse genutzt, enthält das Gasgemsich neben CO und H₂ auch CO₂. Es können aber auch beispielsweise CO mit aus einer Elektrolyse angereichertem Wasserstoff als Synthesegas verwendet werden.

Dabei kann vorgesehen sein, dass der Recycle-Strom an verschiedenen Abschnitten in den Prozess zurückgeführt wird, wobei unter dem Prozess das Verfahren zur Herstellung von Methan aus einem Synthesegas zu verstehen ist. Vorteilhafterweise wird der Recycle-Strom in den Synthesegasverdichter zurückgeführt. Denkbar ist aber auch, dass der Recycle-Strom direkt in die erste und/oder zweite Methansynthese rückgeführt wird. Auch eine Kombination der Rückführungen ist denkbar, sodass die Rückführung in den Synthesegasverdichter und/oder die erste Methansynthese und/oder die zweite Methansynthese zurückgeführt wird.

Um höhere Reinheiten zu erzielen, kann eine CO₂-Abtrennung als auch eine H₂-Entfernung, beziehungsweise eine Entfernung von nicht umgesetzten Edukten. Bei der erfindungsgemäßen Lösung kann eine Anpassung beziehungsweise eine Modifikation eines bestehenden Prozesses erfolgen, bei dem die gewünschten Reinheiten erreicht werden können, wobei gleichzeitig Stoffe, die nicht im Endprodukt enthalten sein sollen, entfernt und im Prozess wiederverwendet werden können. Die Zugabe der Gasströme aus der Methanaufbereitung kann vor oder nach dem Synthesegaskompressor (oder in eine Kompressor-Zwischenstufe) oder auch zwischen zwei Reaktorstufen erfolgen. Je nach Konzept und Aufreinigungstechnologie-kann ein zusätzlicher Kompressor zur Komprimierung der Recycleströme notwendig werden.

Weitere bevorzugte Ausgestaltungen der Erfindung ergeben sich aus den übrigen, in den Unteransprüchen genannten Merkmalen.

Bei einer ersten Ausgestaltung des erfindungsgemäßen Verfahrens ist vorgesehen, dass die Aufbereitung des Produktstroms eine Dehydrierung umfasst. Durch die Dehydrierung kann Wasser aus dem feuchten Methanstrom entfernt werden. Dabei fällt bevorzugt nicht ausschließlich Wasser als Strom an, sondern ein wasserbeladener Methanstrom.

Bei einer weiteren Ausgestaltung des erfindungsgemäßen Verfahrens ist vorgesehen, dass nach der Dehydrierung eine CO₂-Abscheidung erfolgt und dass das abgetrennte CO₂ dem Synthesegasverdichter zugeführt wird. Für die CO₂-Entfernung sind ad- und absorptive Verfahren denkbar. Für größere Kapazitäten bietet sich ein absorptives Verfahren an. Dabei ist sowohl eine chemische als auch eine physikalische Wäsche denkbar. Bei der Wäsche fällt ein CO₂-reicher Strom an, der zur Methan-Synthesezurückgeführt werden kann. Bei einem adsorptiven Verfahren wird CO₂ zunächst gebunden und anschließend bei der Regeneration des Adsorbens mit einem Trägergas freigesetzt. Als Trägergas kann Methan eingesetzt werden. Wird ein Teilstrom des zuvor aufgereinigten Methans abgetrennt und für die Regeneration eingesetzt, kann das CO₂-beladene Gas zurück zur Methan-Synthese geleitet werden und das CO₂ erneut der Methanbildung zugeführt werden. Auch wenn die Regeneration ein diskontinuierlicher Prozess ist, bietet es sich an Methan kontinuierlich abzutrennen und entweder direkt zurückzuführen oder zunächst zur Regeneration zu verwenden. Das abgetrennte Methan kann sowohl für die Regeneration des Adsorbens in der Trocknung als auch in der CO₂-Abtrennung eingesetzt werden. Das Wasser aus der Trocknung sollte aber zunächst abgetrennt. werden, bevor es zur Synthese zurückgeführt wird.

Zusätzlich oder alternativ ist bei einer weiteren Ausgestaltung des erfindungsgemäßen Verfahrens vorgesehen, dass nach der CO₂-Abscheidung eine Wasserstoffrückgewinnung erfolgt und dass der rückgewonnene Wasserstoff dem Synthesegasverdichter zugeführt wird. Für eine Wasserstoffabtrennung bieten sich prinzipiell zwei Verfahren an, die Membrantechnologie und die Druckwechseladsorption (PSA). Bei beiden Verfahren fällt ein Wasserstoff-reicher Strom an. Bei einer PSA fällt der Wasserstoffstrom. bei einem erhöhten Druck an, während bei der Membran der Wasserstoffstrom bei einem reduzierten Druck anfällt und eventuell wieder verdichtet werden muss, je nachdem, wie der abgetrennte Wasserstoff weiter verwendet werden soll.

Darüber hinaus ist bei einer weiteren Ausgestaltung des erfindungsgemäßen Verfahrens vorgesehen, dass bei der Dehydrierung ein wasserbeladener Methanstrom anfällt, wobei der wasserbeladene Methanstrom in Strömungsrichtung nach der zweiten Methansynthese rückgeführt wird. In diesem Fall kann der wasserbeladene Methanstrom im Bereich des Hochdruckabscheiders rückgeführt werden, sodass Wasser aus dem Strom abgeschieden werden kann.

Bei einer weiteren Ausgestaltung des erfindungsgemäßen Verfahrens ist vorgesehen, dass bei der Dehydrierung ein wasserbeladener Methanstrom anfällt, dass das Wasser in einem Wasserabscheider von dem wasserbeladenen Methanstrom abgetrennt wird und dass der resultierende wasserarme Methanstrom als Recycle-Strom dem Synthesegasverdichter zugeführt wird. Wenn kein oder wenig Wasser in dem wasserbeladenen Methanstrom vorhanden ist, kann der Strom auch in die Synthese zurückgeführt werden. Da auch weitere Komponenten wie CO, CO₂ und/oder Wasserstoff in dem wasserbeladenen Methanstrom vorhanden sein können, können diese Edukte weiter in der Synthese reagieren.

Bei einer weiteren vorteilhaften Ausgestaltung des erfindungsgemäßen Verfahrens ist vorgesehen, dass das im ersten Hochdruckabscheider und im zweiten Hochdruckabscheider anfallende Wasser einem Niederdruckabscheider zugeführt wird, wobei in dem Niederdruckabscheider Wasser und ein Restgas anfallen.

Zusätzlich kann bei einer weiteren Ausgestaltung des erfindungsgemäßen Verfahrens vorgesehen sein, dass das Restgas dem Synthesegasverdichter zugeführt wird. Auch das Restgas kann werthaltige Eduktstoffe enthalten. Da das Restgas praktisch wasserfrei ist, kann es auch direkt in die Synthese rückgeführt werden. Durch den Druckunterschied muss es vorab verdichtet werden, sodass eine Rückführung in den Synthesegasverdichter beziehungsweise in eine Stufe des Synthesegasverdichters erforderlich ist.

Bei einer weiteren Ausgestaltung der Erfindung ist vorgesehen, dass das im Niederdruckabscheider angefallene Wasser einer Wasseraufbereitung zugeführt wird und als Prozesswasser verwendet wird. Auf diese Weise kann das Wasser aufbereitet und weiterverwendet werden und wird nicht aus dem Prozess entfernt. Gerade Prozesswasser, als reines Wasser, ist in chemischen Prozessen ein werthaltiges Gut. Durch die Aufbereitung können insgesamt Kosten gespart werden, die ansonsten für frisches Prozesswasser erforderlich wären.

Bei einer weiteren vorteilhaften Ausgestaltung des erfindungsgemäßen Verfahrens ist vorgesehen, dass zur Bereitstellung von Wasserstoff eine Wasserelektrolyse eingesetzt wird und dass das Prozesswasser der Wasserelektrolyse zugeführt wird. Somit kann durch eine strombetriebene Wasserelektrolyse Wasserstoff hergestellt werden, der für die Reaktion genutzt wird. Gleichzeitig wird der Wasserstoff aus dem aufbereiteten Prozesswasser gewonnen, sodass auch weiterhin kein unnötiger Verlust entsteht. Der in der Wasserstoffelektrolyse anfallende Sauerstoff kann aufgefangen und ebenfalls als Wertprodukt verkauft oder im Prozess zur Befeuerung von Wärmequellen genutzt werden.

Die vorgenannte Aufgabe wird außerdem gelöst von einer Anlage zur Herstellung von Methan aus einem Synthesegas. Es ist vorgesehen, dass die Anlage mindestens die folgenden Komponenten umfasst: einen Synthesegasverdichter zum Verdichten eines Gemisches umfassend Wasserstoff und CO₂; eine erste Methansynthese zum Umsetzen des Gemisches; einen ersten Hochdruckabscheider, zum Abscheiden von Wasser von dem Produktstrom der ersten Methansynthese; eine zweite Methansynthese; einen zweiten Hochdruckabscheider, zum Abscheiden von Wasser von dem Produktstrom der zweiten Methansynthese; eine Einrichtung zur Aufbereitung des Produktstroms zum Erhalt von Methan und eines Recycle-Stroms; eine Rückführung, die in strömungstechnischer Verbindung mit der Einrichtung zur Aufbereitung des Produktstroms und einem Abschnitt der Anlage steht. Die Anlage kann dazu ausgestaltet sein, ein erfindungsgemäßes Verfahren durchzuführen. Die vorherigen Ausführungen betreffend das erfindungsgemäße Verfahren gelten entsprechend auch für die erfindungsgemäße Anlage.

Bei einer ersten Ausgestaltung der erfindungsgemäßen Anlage ist vorgesehen, dass die Einrichtung zur Aufbereitung des Produktstroms eine Dehydrierung umfasst.

Bei einer weiteren Ausgestaltung der erfindungsgemäßen Anlage ist nach der Dehydrierung eine Einrichtung zur CO₂-Abscheidung vorgesehen, wobei die Einrichtung zur CO₂-Abscheidung strömungstechnisch mit dem Synthesegasverdichter in Verbindung steht.

Ferner ist bei einer weiteren Ausgestaltung der erfindungsgemäßen Anlage nach der Einrichtung zur CO₂-Abscheidung eine Einrichtung zur Wasserstoffrückgewinnung vorgesehen, wobei die Einrichtung zur Wasserstoffrückgewinnung strömungstechnisch mit dem Synthesegasverdichter in Verbindung steht.

Bei einer weiteren vorteilhaften Ausgestaltung der Erfindung ist vorgesehen, dass die Dehydrierung eine weitere Rückführung aufweist, die in Strömungsrichtung nach der zweiten Methansynthese angebunden ist.

Zusätzlich oder alternativ ist bei einer weiteren Ausgestaltung der erfindungsgemäßen Anlage vorgesehen, dass bei der Dehydrierung ein wasserbeladener Methanstrom anfällt, dass ein Wasserabscheider vorgesehen ist und dass der Wasserabscheider strömungstechnisch mit dem Synthesegasverdichter verbunden ist.

Die verschiedenen in dieser Anmeldung genannten Ausführungsformen der Erfindung sind, sofern im Einzelfall nicht anders ausgeführt, mit Vorteil miteinander kombinierbar.

Die Erfindung wird nachfolgend in Ausführungsbeispielen anhand der zugehörigen Zeichnungen erläutert. Es zeigen:
- Figur 1: eine schematische Darstellung eines Ausführungsbeispiels einer erfindungsgemäßen Anlage zur Herstellung von Methan aus einem Synthesegas in einer Blockdarstellung,
- Figur 2: einen Teil der Methanaufbereitung eines Ausführungsbeispiels einer erfindungsgemäßen Anlage zur Herstellung von Methan aus Synthesegas in einer Blockdarstellung,
- Figur 3: einen Teil der Methanaufbereitung mit zwei Rückführungen eines weiteren Ausführungsbeispiels einer erfindungsgemäßen Anlage zur Herstellung von Methan aus Synthesegas in einer Blockdarstellung und
- Figur 4: ein weiteres Ausführungsbeispiel eines Teils der Methanaufbereitung einer erfindungsgemäßen Anlage zur Herstellung von Methan aus Synthesegas mit einer Wasserstoffrückgewinnung in einer Blockdarstellung.

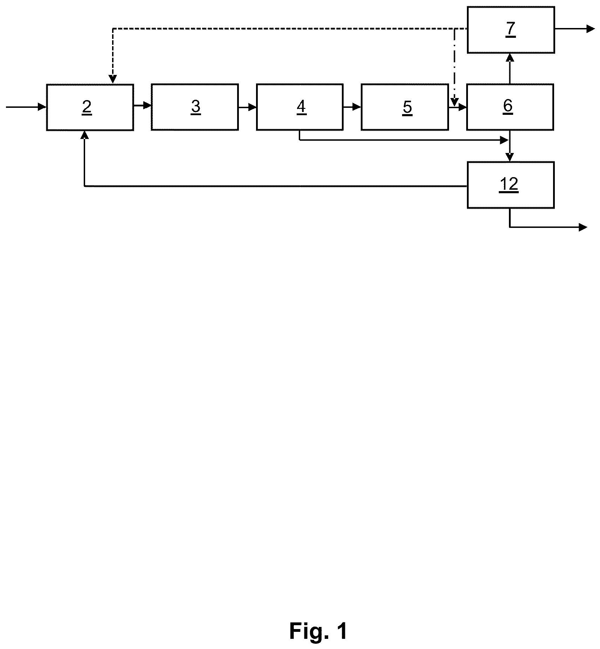

Figur 1 zeigt eine schematische Darstellung einer Anlage 1 zur Herstellung von Methan aus einem Synthesegas. Dabei ist ein Synthesegasverdichter 2 vorgesehen, um ein Gemisch umfassend Wasserstoff und CO₂ zu verdichten. Nach der Verdichtung wird das Gemisch in einer ersten Methansynthese 3 umgesetzt. Der ersten Methansynthese 3 ist ein erster Hochdruckabscheider 4 nachgeschaltet, der Wasser von dem Produktstrom der ersten Methansynthese 3 abscheidet. Im Anschluss wird der Produktstrom einer zweiten Methansynthese 5 zugeführt. Ferner ist der zweiten Methansynthese 5 ein zweiter Hochdruckabscheider 6 nachgeschaltet, um Wasser von dem Produktstrom der zweiten Methansynthese 5 abzuscheiden.

Der Rohmethanstrom, der den zweiten Hochdruckabscheider 6 verlässt, wird in eine Einrichtung 7 zur Aufbereitung des Produktstroms geleitet, um Methan und einen Recycle-Strom zu erhalten. Der Recycle-Strom wird über eine Rückführung 8 in den Prozess zurückgeführt.

Ferner wird das im ersten Hochdruckabscheider 4 und im zweiten Hochdruckabscheider 6 anfallende Wasser einem Niederdruckabscheider 12 zugeführt, wobei in dem Niederdruckabscheider 12 Wasser und ein Restgas anfallen. Das Restgas wird dem Synthesegasverdichter 2 zugeführt. Auch das Restgas kann werthaltige Eduktstoffe enthalten. Da das Restgas praktisch wasserfrei ist, kann es auch direkt in die Synthese rückgeführt werden. Durch den Druckunterschied muss es vorab verdichtet werden, sodass eine Rückführung in den Synthesegasverdichter 2 beziehungsweise in eine Stufe des Synthesegasverdichters 2 erforderlich ist.

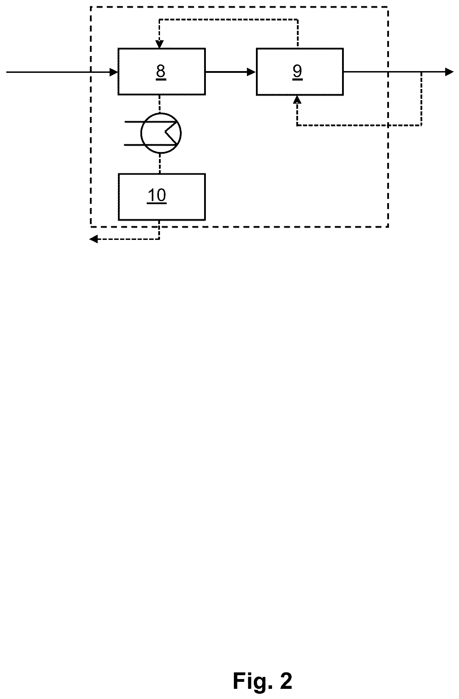

Figur 2 zeigt einen Teil der Einrichtung 7 zur Aufbereitung. Dabei ist vorgesehen, dass die Einrichtung 7 zur Aufbereitung des Produktstroms eine Dehydrierung 8 umfasst. Durch die Dehydrierung 8 kann Wasser aus dem feuchten Methanstrom entfernt werden, wobei nicht ausschließlich Wasser als Strom anfällt, sondern ein wasserbeladener Methanstrom. Ferner ist bei diesem Ausführungsbeispiel nach der Dehydrierung 8 eine CO₂-Abscheidung 9 vorgesehen. Zusätzlich ist ein Wasserabscheider 10 vorgesehen, durch den der Wasserstrom von restlichem Methan befreit werden kann, wobei das Methan wieder in die erste Methansynthese 3 oder die zweite Methansynthese 5 rückgeführt werden kann. Darüber hinaus sind Rückführungen nach der CO₂-Abscheidung 9 zurück in die CO₂-Abscheidung 9 und von der CO₂-Abscheidung 9 in die Dehydrierung 8 vorgesehen.

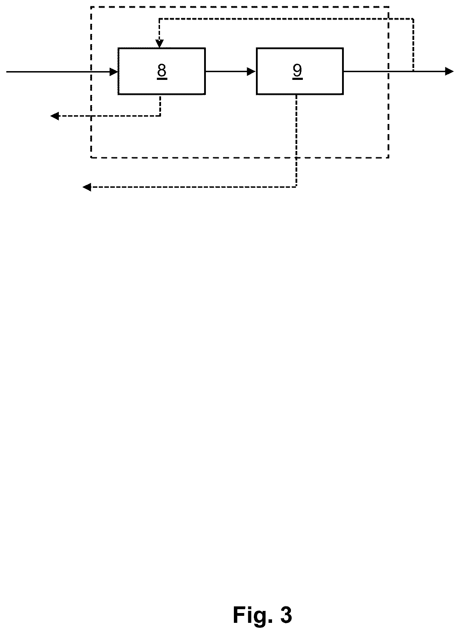

Figur 3 zeigt eine alternative Ausgestaltung der Einrichtung 7 zur Aufbereitung des Rohmethans. Dabei sind entsprechend der Figur 2 ebenfalls eine Dehydrierung 8 und eine CO₂-Abscheidung 9 vorgesehen. Abweichend ist kein Wasserabscheider vorhanden, sodass ein wasserbeladener Methanstrom durch die Dehydrierung 8 erhalten wird. Zusätzlich kann ein aus der CO₂-Abscheidung 9 erhaltener CO₂ haltiger Recycle-Strom in die Methansynthese zurückgeführt werden.

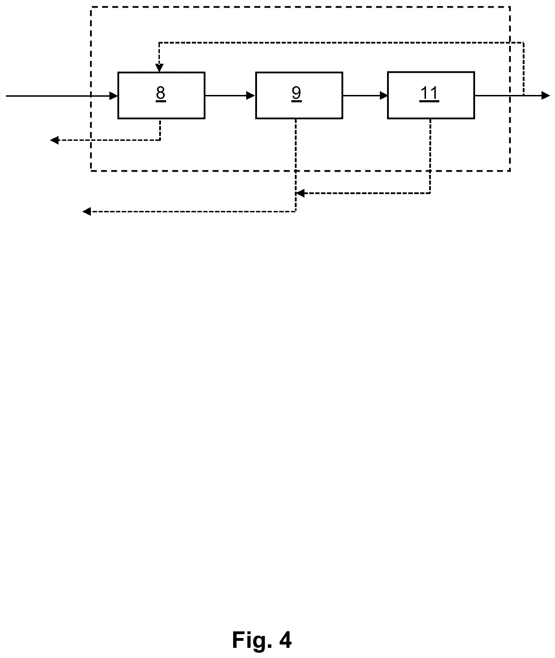

Figur 4 zeigt ein weiteres Ausführungsbeispiel, ähnlich zu dem Ausführungsbeispiel gemäß Figur 3, wobei zusätzlich nach der CO₂-Abscheidung 9 eine Wasserstoffrückgewinnung 11 erfolgt. Der rückgewonnene Wasserstoff kann dem Synthesegasverdichter zugeführt werden. Für eine Wasserstoffabtrennung bieten sich prinzipiell zwei Verfahren an, die Membrantechnologie und die Druckwechseladsorption (PSA). Bei beiden Verfahren fällt ein Wasserstoff-reicher Strom an. Bei einer PSA fällt der Wasserstoffstrom bei einem erhöhten Druck an, während bei der Membran der Wasserstoffstrom bei einem reduzierten Druck anfällt und eventuell wieder verdichtet werden muss, je nachdem, wie der abgetrennte Wasserstoff weiter verwendet werden soll. Der abgetrennte Wasserstoff kann zusammen mit dem abgetrennten CO₂ in die Methansynthese zurückgeführt werden.

### Bezugszeichenliste

- 1: Anlage
- 2: Synthesegasverdichter
- 3: Erste Methansynthese
- 4: Erster Hochdruckabscheider
- 5: Zweite Methansynthese
- 6: Zweiter Hochdruckabscheider
- 7: Einrichtung zur Aufbereitung
- 8: Dehydrierung
- 9: CO₂-Abscheidung
- 10: Wasserabscheider
- 11: Wasserstoffrückgewinnung
- 12: Niederdruckabscheider

## Patentansprüche

1. Verfahren zur Herstellung von Methan aus einem Synthesegas, umfassend mindestens die folgenden Schritte:
a. Verdichten eines Gemisches umfassend Wasserstoff und CO₂ mit einem Synthesegasverdichter (2),
b. Zuführen des Gemisches in eine erste Methansynthese (3);
c. Zuführen des entstandenen Produktstroms in einen ersten Hochdruckabscheider (4), wobei Wasser von dem Produktstrom abgeschieden wird,
d. Zuführen des Produktstroms in eine zweite Methansynthese (5);
e. Zuführen des Produktstroms in einen zweiten Hochdruckabscheider (6), wobei Wasser von dem Produktstrom abgeschieden wird,
f. Aufbereitung des Produktstroms zum Erhalt von Methan und eines Recycle-Stroms, wobei der Recycle-Strom in den Prozess zurückgeführt wird.

2. Verfahren nach Anspruch 1, **dadurch gekennzeichnet, dass** die Aufbereitung des Produktstroms eine Dehydrierung (8) zur Entfernung von Wasser aus dem Produktstrom umfasst.

3. Verfahren nach Anspruch 2, **dadurch gekennzeichnet, dass** nach der Dehydrierung (8) eine CO₂-Abscheidung (9) erfolgt und dass das abgetrennte CO₂ dem Synthesegasverdichter (2) zugeführt wird.

4. Verfahren nach Anspruch 2 oder 3, **dadurch gekennzeichnet, dass** nach der CO₂-Abscheidung (9) eine Wasserstoffrückgewinnung (11) erfolgt und dass der rückgewonnene Wasserstoff dem Synthesegasverdichter (2) zugeführt wird.

5. Verfahren nach einem der Ansprüche 2 bis 4, **dadurch gekennzeichnet, dass** bei der Dehydrierung (8) ein wasserbeladener Methanstrom anfällt, wobei der wasserbeladene Methanstrom in Strömungsrichtung nach der zweiten Methansynthese (5) rückgeführt wird.

6. Verfahren nach einem der Ansprüche 2 bis 4, **dadurch gekennzeichnet, dass** bei der Dehydrierung (8) ein wasserbeladener Methanstrom anfällt, dass das Wasser in einem Wassersabscheider (10) von dem wasserbeladenen Methanstrom abgetrennt wird und dass der resultierende wasserarme Methanstrom als Recycle-Strom dem Synthesegasverdichter (2) zugeführt wird.

7. Verfahren nach einem der Ansprüche 1 bis 6, **dadurch gekennzeichnet, dass** das im ersten Hochdruckabscheider (4) und im zweiten Hochdruckabscheider (6) anfallende Wasser einem Niederdruckabscheider (12) zugeführt wird, wobei in dem Niederdruckabscheider (12) Wasser und ein Restgas anfallen.

8. Verfahren nach Anspruch 7, wobei das Restgas dem Synthesegasverdichter (2) zugeführt wird.

9. Verfahren nach Anspruch 7 oder 8, **dadurch gekennzeichnet, dass** das im Niederdruckabscheider (12) angefallene Wasser einer Wasseraufbereitung zugeführt wird und als Prozesswasser verwendet wird.

10. Verfahren nach Anspruch 9, **dadurch gekennzeichnet, dass** zur Bereitstellung von Wasserstoff eine Wasserelektrolyse eingesetzt wird und dass das Prozesswasser der Wasserelektrolyse zugeführt wird.

11. Anlage (1) zur Herstellung von Methan aus einem Synthesegas, umfassend mindestens die folgenden Komponenten:
a. einen Synthesegasverdichter (2) zum Verdichten eines Gemisches umfassend Wasserstoff und CO₂,
b. eine erste Methansynthese (3) zum Umsetzen des Gemisches;
c. einen ersten Hochdruckabscheider (4), zum Abscheiden von Wasser von dem Produktstrom der ersten Methansynthese (3),
d. eine zweite Methansynthese (5);
e. einen zweiten Hochdruckabscheider (6), zum Abscheiden von Wasser von dem Produktstrom der zweiten Methansynthese,
f. eine Einrichtung (7) zur Aufbereitung des Produktstroms zum Erhalt von Methan und eines Recycle-Stroms
g. eine Rückführung, die in strömungstechnischer Verbindung mit der Einrichtung zur Aufbereitung des Produktstroms und einem Abschnitt der Anlage steht.

12. Anlage nach Anspruch 11, **dadurch gekennzeichnet, dass** die Einrichtung (7) zur Aufbereitung des Produktstroms eine Dehydrierung (8) umfasst.

13. Anlage nach Anspruch 12, **dadurch gekennzeichnet, dass** nach der Dehydrierung (8) eine Einrichtung zur CO₂-Abscheidung (9) vorgesehen ist und dass die Einrichtung zur CO₂-Abscheidung (9) strömungstechnisch mit dem Synthesegasverdichter (2) in Verbindung steht.

14. Anlage nach Anspruch 12 oder 13, **dadurch gekennzeichnet, dass** nach der Einrichtung zur CO₂-Abscheidung eine Einrichtung zur Wasserstoffrückgewinnung (11) vorgesehen ist und dass die Einrichtung zur Wasserstoffrückgewinnung (11) strömungstechnisch mit dem Synthesegasverdichter (2) in Verbindung steht.

15. Anlage nach einem der Ansprüche 12 bis 14, **dadurch gekennzeichnet, dass** die Dehydrierung (8) eine weitere Rückführung aufweist, die in Strömungsrichtung nach der zweiten Methansynthese (5) angebunden ist.

16. Anlage nach einem der Ansprüche 12 bis 14, **dadurch gekennzeichnet, dass** bei der Dehydrierung (8) ein wasserbeladener Methanstrom anfällt, dass ein Wasserabscheider (10) vorgesehen ist und dass der Wasserabscheider (10) strömungstechnisch mit dem Synthesegasverdichter (2) verbunden ist.
